# EUROPEAN PATENT APPLICATION

(11) **EP 0 634 413 A1**
(43) Date of publication of application: **18.01.1995**
(21) Application number: 94110578.5
(22) Date of filing: 07.07.1994
(51) Int. Cl.: C07D 471/04, C07D 401/06, A01N 43/90

(54) **Herbicides**

(30) Priority: 13.07.1993 GB 9314412
(71) Applicant: RHONE POULENC AGRICULTURE LTD., Ongar, Essex CM5 OHW (GB)
(72) Inventor: Haylor, Barry David G., Ongar, Essex (GB); Smith, Philip Henry G., Ongar, Essex (GB)
(74) Representative: Brachotte, Charles

(57) **Abstract**

The invention provides compounds of formula: 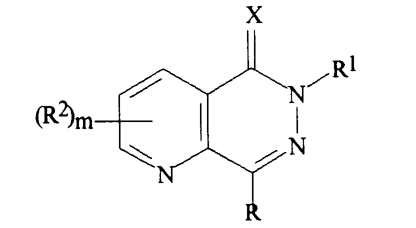
wherein R and R¹ independently represent hydrogen, optionally halogenated alkyl alkenyl or alkynyl;
-(-CR³R⁴)ₙ-(optionally subtituted phenyl); -(-CR³R⁴)ₙHet; or
-(-CR³R⁴)ₙ-Ar, wherein Ar is an optionally substituted bicycle;
wherein at least one of the groups R and R¹ represents -(-CR³R⁴)ₙ-Ar;
R² represents R⁵ or optionally substituted phenyl;
X is oxygen or sulphur; m is 0, 1, 2 or 3;
R³ and R⁴, independently represent hydrogen, alkyl or haloalkyl;
R⁵ is halogen, optionally halogenated alkyl, alkenyl or alkynyl;
or a group selected from cyano, nitro, -CO₂R⁶, -S(O)ₚR⁶, -NR³R⁴, -COR⁶, -S(O)ₚR⁷, -CO₂R⁷, -OR⁷, -CONR³R⁴, -OSO₂R⁷, -OSO₂R⁸, -OCH₂R⁷, -N(R³)COR⁸, -N(R³)SO₂R⁸, -N(R³)SO₂R⁷, -SO₂NR³R⁴, -Si(R⁸)₃ and -OR⁶;
n is zero, 1 or 2; q is zero or an integer from 1 to 5;
Het represents an optionally substituted heterocycle;
R⁶ represents hydrogen, alkyl or haloalkyl;
p is zero, one or two; r is one or two;
R⁷ represents optionally substituted phenyl;
R⁸ represents alkyl or haloalkyl;
R⁶¹ and R⁶² independently represent hydrogen, halogen, alkyl or haloalkyl;
and agriculturally acceptable salts thereof;
which possess utility as herbicides.

## Description

This invention relates to novel pyrido[2,3-d]pyridazin-5-one and pyrido[2,3-d]pyridazin-5-thione derivatives, processes for their preparation, compositions containing them and their use as herbicides.

WO-A-93/07146 discloses certain pyridopyridazinones having pharmacetical activity and in particular names certain 8-(3,4-methylenedioxyphenyl)pyrido[2,3-d]pyridazin-5-ones. No suggestion that these compounds possess pesticidal activity is found in this document.

The present invention provides pyrido[2,3-d]pyridazin-5-one and pyrido[2,3-d]pyridazin-5-thione derivatives of formula (I): 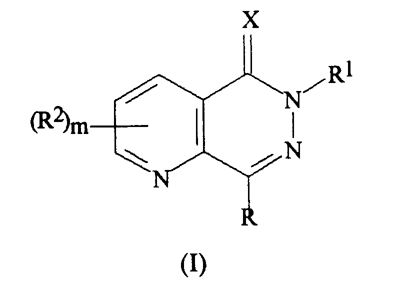
wherein:-
R and R¹, which may be the same or different, each represents:-
the hydrogen atom,
a straight- or branched chain alkyl, alkenyl or alkynyl group containing up to six carbon atoms optionally substituted by one or more halogen atoms;
a group -(-CR³R⁴)ₙ-(phenyl)-(R⁵)_{q};
a group -(-CR³R⁴)ₙHet;
a group -(CR³R⁴)ₙ-Ar, wherein Ar represents phenyl or pyridyl optionally substituted by one or more groups R⁵ and wherein two substituents on adjacent positions of the ring, together with the two atoms to which they are attached, form a 5- to 7-membered alicyclic ring (which is optionally unsaturated) or an aromatic ring, optionally containing one or more heteroatoms (preferably selected from oxygen, sulphur and nitrogen, it being understood that a sulphur atom, where present, may be in the form of a group -SO- or -SO₂-), wherein the alicyclic or aromatic ring is optionally substituted by one or more groups R⁵¹ which may be the same or different;
provided that at least one of the groups R and R¹ represents -(-CR³R⁴)ₙ-Ar;
R² represents:-
a group R⁵;
or phenyl optionally substituted by from one to five groups R⁵ which may be the same or different;
X represents oxygen or sulphur;
m represents zero or an integer from one to three;
where m is greater than one the groups R² may be the same or different;
R³ and R⁴, which may be the same or different, each represents the hydrogen atom or a straight- or branched- chain alkyl group containing up to four carbon atoms optionally substituted by one or more halogen atoms;
R⁵ represents:-
a halogen atom;
a straight- or branched- chain alkyl, alkenyl or alkynyl group containing up to six carbon atoms optionally substituted by one or more halogen atoms; or
a group selected from cyano, nitro, -CO₂R⁶, -S(O)ₚR⁶, -NR³R⁴, -COR⁶, -S(O)ₚR⁷, -CO₂R⁷, -OR⁷, -CONR³R⁴, -OSO₂R⁷, -OSO₂R⁸, -OCH₂R⁷, -N(R³)COR⁸, -N(R³)SO₂R⁸, -N(R³)SO₂R⁷, -SO₂NR³R⁴, -Si(R⁸)₃ and -OR⁶;
n represents zero, one or two; where n is two the groups -(-CR³R⁴)- may be the same or different;
q represents zero or an integer from one to five; where q is greater than one the groups R⁵ may be the same or different;
R⁵¹ is as hereinbefore defined for R⁵ or represents =O, = S or a group -O(-CR⁶¹R⁶²-)ᵣ-O-;
Het represents a 5- or 6- membered heterocycle containing from 3 to 5 carbon atoms in the ring and from 1 to 3 heteroatoms in the ring selected from nitrogen, sulphur and oxygen (e.g. pyridyl, pyrimidyl, thienyl or pyrazolyl) optionally substituted by one or more groups R⁵ which may be the same or different;
R⁶ represents the hydrogen atom or a straight- or branched-chain alkyl group containing up to six carbon atoms optionally substituted by one or more halogen atoms;
p represents zero, one or two; r represents one or two; where r is two the groups -(-CR⁶¹R⁶²)- may be the same or different;
R⁷ represents phenyl optionally substituted by from one to five groups which may be the same or different selected from halogen, nitro, cyano, R⁶ and -OR⁶;
R⁸ represents a straight- or branched-chain alkyl group containing up to six carbon atoms optionally substituted by one or more halogen atoms;
R⁶¹ and R⁶², which may be the same or different, each represents a hydrogen or halogen atom or a straight- or branched-chain alkyl group containing up to four carbon atoms optionally substituted by one or more halogen atoms;
and agriculturally acceptable salts thereof.

Compounds in which X represents oxygen and R¹ represents hydrogen may exist in enolic tautomeric forms. Furthermore, in certain cases, the substituents R, R¹, R², R³, R⁴, R⁵, R⁵¹, R⁶, R⁶¹, R⁶² and R⁸ contribute to optical and/or stereoisomerism. All such forms are embraced by the present invention.

By the term "agriculturally acceptable salts" is meant salts the cations or anions of which are known and accepted in the art for the formation of salts for agricultural or horticultural use. Preferably the salts are water soluble. Suitable salts formed by compounds of formula I which are acidic, e.g. compounds containing a carboxy group, with bases include alkali metal (e.g. sodium and potassium) salts, alkaline earth metal (e.g. calcium and magnesium) salts and ammonium (e.g. diethanolamine, triethanolamine, octylamine, dioctylamine and morpholine) salts.

Suitable acid addition salts, e.g. formed by compounds of formula I containing an amino group, include salts with inorganic acids, for example hydrochlorides, sulphates, phosphates and nitrates and salts with organic acids, for example acetic acid.

It is to be understood that where reference is made in the specification to the compounds of formula I, such reference is intended to include salts where the context so permits.

Where the group Ar represents optionally substituted phenyl or pyridyl with two substituents on adjacent positions of the ring forming a 5- to 7- membered alicyclic ring (which is optionally unsaturated) or an aromatic ring, which contains one or more heteroatoms in the ring, generally there will be from one to three heteroatoms. Examples of the group Ar include optionally substituted methylenedioxybenzene, 2-mercaptobenzimidazole, 2-hydroxybenzimidazole, 2,3-dihydroberzofuran, 1,3-benzoxathiazole, 1,2-benzoxathiazole, (3H)-1,2-benzisothiazole-1,1-dioxide, 1,2,5-benzothiadiazole- 1,1-dioxide, indoline, benzofuroxan and 2,3-dihydrobenzo[b]thiophene.

Furthermore, where Ar represents pyridyl optionally substituted by one or two groups R⁵, the two substituents on adjacent atoms of the pyridyl ring which form a 5- to 7- membered alicyclic or aromatic ring may be attached to two carbon atoms or to a carbon and nitrogen atom of the pyridyl ring.

A number of these compounds are novel and the invention accordingly provides compounds of formula (I) as hereinbefore defined with the proviso that when X is oxygen, m is zero and R represents 3,4-methylenedioxyphenyl, R¹ is not selected from the group consisting of hydrogen, phenyl, benzyl, ethyl and 4-pyridylmethyl.

A preferred class of compounds of formula (I) because of their herbicidal properties are those wherein:-
R represents a group -(-CR³R⁴)ₙ-Ar, wherein Ar represents phenyl optionally substituted by from one to three groups R⁵ and wherein two substituents in the 2,3- or 3,4- positions of the ring, together with the two atoms to which they are attached, form a 5- or 6- membered alicyclic ring (which is optionally unsaturated) or an aromatic ring, containing one or two heteroatoms selected from oxygen, sulphur and nitrogen (e.g. benzothiazole, benzoxazole, methylenedioxybenzene, benzimidazole, indole or indazole), wherein the alicyclic or aromatic ring is optionally substituted by one or more groups R⁵¹ which may be the same or different;
R¹ represents phenyl optionally substituted by from one to five groups R⁵ which may be the same or different;
R² represents:-
a halogen atom;
a straight- or branched- chain alkyl, alkenyl or alkynyl group containing up to six carbon atoms optionally substituted by one or more halogen atoms; or
a group selected from cyano, nitro, -CO₂R⁶, -S(O)ₚR⁶, -NR³R⁴, -COR⁶ and -OR⁶;
n represents zero;
R⁵¹ represents:-
a halogen atom;
a straight- or branched- chain alkyl group containing up to six carbon atoms optionally substituted by one or more halogen atoms;
a group selected from cyano, nitro, -CO₂R⁶, -S(O)ₚR⁶, -NR³R⁴, -COR⁶, -S(O)ₚR⁷, -CO₂R⁷, -OR⁷, -CONR³R⁴, -OSO₂R⁷ and -OR⁶; and
X represents oxygen.

Where m is one preferably R² occupies the 3-position of the pyridyl ring.

A particularly preferred class of compounds of formula (I) because of their herbicidal properties are those wherein:-
R is 2,3-methylenedioxyphenyl, wherein the methylene group is optionally substituted by one or two (preferably two) groups R⁵¹ which may be the same or different (preferably the same);
R¹ represents phenyl optionally substituted in the 3- and/or 4-position by R⁵;
R² represents:-
a halogen atom;
a straight- or branched chain alkyl group containing up to four carbon atoms optionally substituted by one or more halogen atoms (e.g. methyl);
or a group selected from -S(O)ₚR⁶, -NR³R⁴ and -OR⁶;
R⁵¹ represents:-
a halogen atom (preferably fluorine); or
a straight- or branched chain alkyl group containing up to four carbon atoms optionally substituted by one or more halogen atoms;
R⁵ represents:-
a halogen atom (preferably fluorine or chlorine); or
a straight- or branched chain alkyl group containing up to four carbon atoms optionally substituted by one or more halogen atoms;
or a cyano group;
m is zero, one or two; and X represents oxygen.

A further particularly preferred class of compounds of formula (I) because of their herbicidal properties are those wherein:-
R and R¹, which may be the same or different, each represents:-
phenyl substituted in the 3- and/or 4- position by R⁵;
2,3- or 3,4-methylenedioxyphenyl, wherein the methylene group is optionally substituted by two fluorine atoms;
provided that at least one of the groups R and R¹ is 2,3- or 3,4-methylenedioxyphenyl, wherein the methylene group is optionally substituted by two fluorine atoms;
R² represents a halogen atom;
R⁵ represents a halogen atom, trifluoromethyl or cyano;
m represents zero or one; and
X represents oxygen.

Particularly important compounds of formula (I) include the following:
1. 8-(2,3-difluoromethylenedioxyphenyl)-6-(4-fluorophenyl)pyrido[2,3-d]pyridazin-5-one;
2. 6-(4-chlorophenyl)-8-(2,3-difluoromethylenedioxyphenyl)pyrido[2,3-d]pyridazin-5-one;
3. 8-(2,3-difluoromethylenedioxyphenyl)-6-(4-trifluoromethylphenyl)pyrido[2,3-d]pyridazin-5-one;
4. 8-(3,4-difluoromethylenedioxyphenyl)-6-(4-fluorophenyl)pyrido[2,3-d]pyridazin-5-one;
5. 6-(4-fluorophenyl)-8-(2,3-methylenedioxyphenyl)pyrido[2,3-d]pyridazin-5-one;
6. 6-(3,4-difluoromethylenedioxyphenyl)-8-(3-trifluoromethylphenyl)pyrido[2,3-d]pyridazin-5-one;
7. 3-chloro-8-(2,3-difluoromethylenedioxyphenyl)-6-(4-fluorophenyl)pyrido[2,3-d]pyridazin-5-one;
8. 3-chloro-8-(2,3-difluoromethylenedioxyphenyl)-6-(4-trifluoromethylphenyl)pyrido[2,3-d]pyridazin-5-one;
9. 6-(3-cyano-4-fluorophenyl)-8-(2,3-difluoromethylenedioxyphenyl)pyrido[2,3-d]pyridazin-5-one;
10. 8-(2,3-difluoromethylenedioxyphenyl)-6-(4-fluoro-3-trifluoromethylphenyl)pyrido[2,3-d]pyridazin-5-one;
11. 6-(4-cyanophenyl)-8-(2,3-difluoromethylenedioxyphenyl)pyrido[2,3-d]pyridazin-5-one;
12. 8-(2,3-difluoromethylenedioxyphenyl)-6-(3,4-difluorophenyl)pyrido[2,3-d]pyridazin-5-one;
13. 8-(2,3-difluoromethylenedioxyphenyl)-3-fluoro-6-(4-fluorophenyl)pyrido[2,3-d]pyridazin-5-one; and
14. 8-(2,3-difluoromethylenedioxyphenyl)-3-fluoro-6-(4-trifluoromethylphenyl)pyrido[2,3-d]pyridazin-5-one.

The numbers 1 to 14 are assigned to these compounds for reference and identification hereinafter.

Compounds of formula I may be prepared by the application or adaptation of known methods (i.e. methods heretofore used or described in the literature), for example as hereinafter described.

In the following description, where symbols appearing in formulae are not specifically defined, it is to be understood that they are "as hereinbefore defined" in accordance with the first definition of each symbol in the specification.

It is to be understood that in the description of the following processes the sequences may be performed in different orders, and that suitable protecting groups may be required to achieve the compounds sought.

According to a feature of the present invention compounds of formula I wherein X represents oxygen may be prepared by the reaction of a compound of formula II: 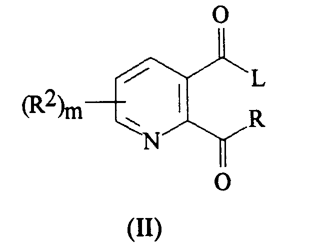
wherein R, R² and m are as hereinbefore defined and L is a leaving group, with a hydrazine of formula III or a salt thereof:

R¹-NHNH₂ (III)

wherein R¹ is as hereinbefore defined. Generally L is -OH, straight- or branched- chain alkoxy containing up to 4 carbon atoms (e.g. ethoxy), or halogen, for example chlorine. The reaction is generally carried out in a solvent such as toluene or ethanol. Where the hydrazine of formula III is used in the form of a salt (such as the hydrochloride) the reaction is generally performed in the presence of a base or acid acceptor such as triethylamine or potassium carbonate. The reaction is generally performed from room temperature to the reflux temperature of the mixture and preferably with azeotropic removal of water from the mixture. A number of the compounds of formula II are novel and as such constitute a further feature of the present invention.

According to a further feature of the present invention compounds of formula I in which X represents oxygen may be prepared by the cyclisation of a compound of formula IIa: 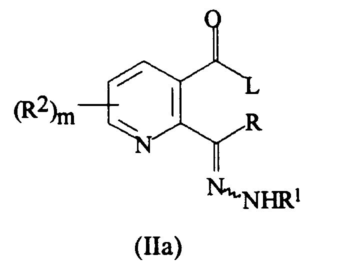
wherein R, R¹, R², m and L are as hereinbefore defined. The reaction is generally carried out in a alcoholic solvent such as methanol or ethanol in the presence of a base or acid acceptor such as triethylamine or potassium carbonate optionally in the presence of a catalyst, for example para-toluenesulphonic acid. The reaction is generally performed at a temperature from room temperature to the reflux temperature of the mixture and preferably with azeotropic removal of water from the mixture.

A number of the compounds of formula IIa are novel and as such constitute a further feature of the present invention.

According to a further feature of the present invention compounds of formula I wherein X represents sulphur may be prepared from corresponding compounds of formula I in which X represents oxygen by reaction with a thionation reagent to convert the carbonyl group to a thiocarbonyl group. Suitable thionation reagents include Lawessons' Reagent, i.e. [2,4-bis(4-methoxyphenyl)-1,3-dithia-2,4-diphosphetane-2,4-disulphide], and phosphotus pentasulphide. The reaction is generally carried out in a suitable solvent, for example toluene, at a temperature from 50^{o}C to the reflux temperature of the mixture.

Intermediates in the preparation of compounds of formula I may be prepared by the application or adaptation of known methods.

Compounds of formula II wherein L is OH or straight- or branched- chain alkoxy containing up to 4 carbon atoms may be prepared by the oxidation of a compound of formula IV: 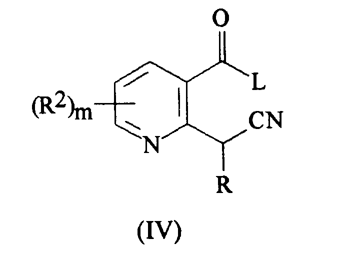
wherein R, R² and m are as hereinbefore defined, to convert the cyano-methylene group to a carbonyl group. The reaction is carried out in the presence of a base, for example lithium diisopropylamide, potassium carbonate or sodium hydride, in an anhydrous solvent, for example dimethyl sulphoxide, tetrahydrofuran (THF), 1,4-dioxane or acetonitrile, at temperatures from -72^{o}C to the reflux temperature of the mixture. Generally the oxidant used is air or oxygen.

Alternatively, the reaction may be carried out in a two-phase system comprising an organic solvent such as toluene or dichloromethane and an aqueous solution of a base, for example sodium hydroxide, in the presence of a quaternary ammonium salt, for example triethyl benzylammonium chloride. Generally the oxidant used is air or oxygen. The reaction is generally performed at a temperature from room temperature to the reflux temperature of the mixture.

Compounds of formula II wherein L is straight- or branched-chain alkoxy containing up to 4 carbon atoms and R is hydrogen may also be prepared by the reduction of the corresponding pyridine dicarboxylate of formula II in which R is replaced by OH, for example as described by Queguiner et al., Bull. Soc. Chim. Fr., 1969, 3678.

Compounds of formula II in which L is straight- or branched-chain alkoxy containing up to 4 carbon atoms or halogen may be prepared from the corresponding carboxylic acid of formula II in which L represents -OH by the application or modification of known methods.

Compounds of formula IIa in which L is hydroxy may be prepared by the reaction of a compound of formula II wherein R, R² and m are as hereinbefore defined with a compound of formula III wherein R¹ is as hereinbefore defined. The reaction is generally performed in a solvent such as ethanol or methanol. The reaction is generally carried out at a temperature from ambient to the reflux temperature of the solvent.

Compounds of formula IIa in which L is not hydroxy may be prepared by the reaction of a compound of formula II wherein R, R² and m are as hereinbefore defined with a compound of formula III wherein R¹ is as hereinbefore defined, in the presence of a Lewis acid (preferably titanium tetrachloride). The reaction is generally performed in a solvent such as hexane or toluene and at a temperature from -20^{o}C to the reflux temperature of the mixture. This reaction is particularly useful for preparing compounds of formula IIa where L is straight- or branched- chain alkoxy having up to 4 carbon atoms, and the general method is described in the literature (e.g. J. Organic Chem. 1967, Volume 32, p3246).

Certain compounds of formula III may be prepared by diazotisation and reduction of a compound of formula R¹NH₂. Diazotisation is typically carried out using sodium nitrite and hydrochloric acid or sulphuric and acetic acids. The temperature of the reaction is generally between 0^{o} and 80^{o}C. The reducing agent is, for example tin (II) chloride and the solvent is hydrochloric acid.

Compounds of formula III may also be prepared by reacting hydrazine hydrate with a suitable compound of formula R¹-Y⁴, wherein Y⁴ is chlorine or fluorine, for example where R¹ represents pyridine or an aromatic ring activated by an electron withdrawing group, for example trifluoromethyl. The reaction is performed in a solvent, for example ethoxyethanol at a temperature from 60^{o}C to reflux temperature of the solvents.

Compounds of formula IV in which L is straight- or branched-chain alkoxy containing up to 4 carbon atoms, or preferably -OH, may be prepared by the reaction between a nicotinic acid derivative of formula V: 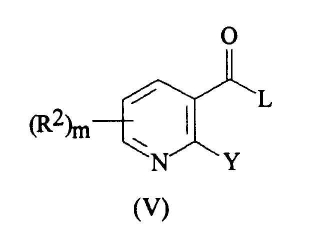
wherein R² and m are as hereinbefore defined and Y is a leaving group, for example halogen, and a nitrile of formula VI:

RCH₂CN (VI)

wherein R is as hereinbefore defined. The reaction is performed in the presence of a base, for example sodium hydride, sodium amide or an alkali metal alkoxide in a solvent, for example toluene, 1,4-dioxane or THF, at temperatures between 0^{o}C and the reflux temperature of the solvent. The reaction may be optionally performed in the presence of a phase transfer catalyst, for example tris[2-(2-methoxyethoxy)ethyl]amine (commonly known as TDA-1).

Compounds of formula IV in which L is -OH or straight- or branched- chain alkoxy containing up to 4 carbon atoms and R represents a straight- or branched- chain alkyl, alkenyl or alkynyl group containing up to six carbon atoms optionally substituted by one or more halogen atoms, or a group selected from -[CR³R⁴]ₙ-(phenyl)-(R⁵)_{q}, -[CR³R⁴]ₙ-Ar and -[CR³R⁴]ₙ-Het, wherein n is one or two, may be prepared by the reaction of a 2-cyanomethylnicotinic acid derivative of formula VII: 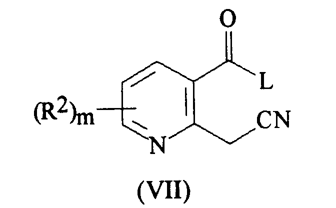
wherein R² and m are as hereinbefore defined and L is -OH or straight- or branched- chain alkoxy containing up to 4 carbon atoms, with a compound of formula VIII:

R-Z (VIII)

wherein R represents a straight- or branched- chain alkyl, alkenyl or alkynyl group containing up to six carbon atoms optionally substituted by one or more halogen atoms, or a group selected from -[CR³R⁴]ₙ-(phenyl)-(R⁵)_{q}, -[CR³R⁴]ₙ-Ar and -[CR³R⁴]ₙ-Het, wherein n is one or two, and Z is a leaving group, for example halogen or tosyl. Where R is a straight- or branched-chain alkyl, alkenyl or alkynyl group containing up to six carbon atoms optionally substituted by one or more halogen atoms, Z is not attached to an unsaturated carbon atom. The reaction is performed in the presence of a base and is widely described in the chemical literature (for example, as described by Masuyama et al., Chem. Lett., 1977,1439).

Compounds of formula VI may be prepared by cyanation of a compound of formula IX:

RCH₂Y¹ (IX)

wherein R is as hereinbefore defined and Y¹ is a leaving group, for example chlorine or bromine. Preferably sodium cyanide is the cyanide source and the reaction is generally performed in the presence of a solvent, for example aqueous ethanol. The reaction temperature is between room temperature and the reflux temperature of the solvent mixture.

Compounds of formula (IX) in which Y¹ is halogen may be prepared by halogenation of a compound of formula RCH₂OH. The halogen source is, for example phosphorus tribromide. The reaction is conducted in a solvent, for example diethyl ether, at a temperature between 0^{o}C and the reflux temperature of the solvent.

Compounds of the formula (IX) wherein Y¹ is chlorine or bromine may also be prepared by halogenation of compounds of the formula RCH₃ by known methods, for example in the presence of a suitable halogen source, for example N-chloro or N-bromosuccinimide in a solvent, e.g. carbon tetrachloride or chloroform, at a temperature between room temperature and the reflux temperature of the solvent. The reaction is preferably conducted in the presence of a radical initiator, for example benzoyl peroxide.

Compounds of formula RCH₂OH may be prepared by reduction of a compound of formula RC(O)Y², wherein Y² is hydrogen, OH or alkoxy. Suitable reducing agents include for example sodium borohydride, lithium aluminium hydride and diisobutyl aluminium hydride and the solvent is, for example ethanol, diethyl ether or tetrahydrofuran. The reaction temperature is generally between 0^{o} and the reflux temperature of the solvent.

Compounds of formulae RCH₂OH and RC(O)Y² may be prepared by the lithiation of suitable compounds of formula RH followed by condensation with paraformaldehyde, dimethylformamide or carbon dioxide. The reaction is carried out in a solvent, for example diethyl ether or THF and the lithiating agent is, for example lithium diisopropylamide in the presence of tetramethylenediamine. This reaction is described in the literature, for example in European Patent No. 333658 or by G Jones et al, J.Chem.Soc.Perkin 1,1982, 967.

Compounds of formulae RCH₂OH and RC(O)Y² may also be prepared by a lithium-halogen exchange reaction on a compound of formula RBr, followed by condensation with paraformaldehyde, dimethylformamide or carbon dioxide. The reaction is carried out using, for example a lithium reagent such as n-butyl lithium, and a solvent such as diethyl ether or THF. The reaction is generally performed between -80^{o} and 0^{o}C.

Compounds of formula RBr may be prepared by bromination of suitable compounds of formula RH using for example bromine and iron powder in a suitable solvent, for example carbon tetrachloride at a temperature between 0^{o}C and the reflux temperature of the solvent.

Compounds of the formula R¹NH₂ may be prepared by reduction of a compound of formula R¹NO₂, for example using tin (II) chloride in hydrochloric acid at a temperature from 0^{o} to 60^{o}C.

Compounds of formula R¹NO₂ may be prepared by the nitration of suitable compounds of the formula R¹H, for example using a mixture of nitric and sulphuric acids at a temperature between ambient and 100^{o}C. This reaction is described in the literature, e.g. European Patent No. 537519.

Compounds of formula R¹NH₂ may also be prepared by the cleavage of a compound of the formula R¹NHC(O)Y³, wherein Y³ is alkyl (preferably t-butyl), for example using iodotrimethylsilane in a solvent, for example acetonitrile at ambient temperature:

Compounds of the formula R¹NHC(O)Y³ may be prepared by rearrangement of an azide of formula R¹C(O)N₃. This is typically achieved by refluxing in a solvent such as toluene, followed by addition of an alcohol (preferably t-butanol) and heating at reflux.

Compounds of formula R¹C(O)N₃ may be prepared by the reaction of an acid of the formula R¹CO₂H with diphenylphosphoryl azide in dimethylformamide and triethylamine at a temperature between 0^{o} and 50^{o}C.

Compounds of formula R¹NH₂ may also be prepared by treating an amide of formula R¹C(O)NH₂ with aqueous sodium hypochlorite and sodium hydroxide. The reaction is generally performed at a temperature from 0^{o} to 80^{o}C.

Compounds of formula R¹C(O)NH₂ and R¹C(O)N₃ may be prepared by the reaction of an acid chloride of the formula R¹COCl with aqueous ammonia or sodium azide respectively.

Acid chlorides of formula R¹C(O)Cl may be prepared by reacting an acid of formula R¹CO₂H with a chlorinating reagent, e.g. thionyl chloride at reflux.

Compounds of formulae V, VII and VIII are known or may be prepared by the application of known methods. Agriculturally acceptable salts of compounds of formula (I) may be prepared by known methods.

The following Examples illustrate the preparation of compounds of formula I and the Reference Examples illustrate the preparation of intermediates. In the present specification m.p. means melting point. Where the letters NMR appear the characteristics of a nuclear magnetic spectra follow. Unless otherwise stated percentages are by weight.

### Example 1

Triethylamine (1.81g) was added to 4-fluorophenylhydrazine hydrochloride (2.91g) in toluene. 2-(2,3-Difluoromethylenedioxybenzoyl)nicotinic acid (4.6g) was added and the mixture stirred at reflux for 4 hours. Water was removed azeotropically from the mixture using a Dean-Stark apparatus. The reaction mixture was then washed with 2N hydrochloric acid and brine. The organic phase was dried then evaporated. The crude product was triturated with diethyl ether/hexane to yield 8-(2,3-difluoromethylenedioxyphenyl)-6-(4-fluorophenyl)pyrido[2,3-d]-pyridazin-5-one (compound 1, 3.43g) as a beige solid, m.p. 173-174^{o}C.

By proceeding similarly the following compounds of formula (I) were prepared:
6-(4-chlorophenyl)-8-(2,3-difluoromethylenedioxyphenyl)pyrido[2,3-d]pyridazin-5-one (compound 2), m.p. 160-161.4^{o}C;
8-(3,4-difluoromethylenedioxyphenyl)-6-(4-fluorophenyl)pyrido[2,3-d]pyridazin-5-one (compound 4), m.p. 176-177.4^{o}C;
6-(4-fluorophenyl)-8-(2,3-methylenedioxyphenyl)pyrido[2,3-d]pyridazin-5-one (compound 5), m.p. 165.4-167.4^{o}C.

### Example 2

5-Chloro-2-(2,3-difluoromethylenedioxybenzoyl)nicotinic acid (2.5g) was added to a stirred suspension of 4-fluorophenylhydrazine hydrochloride (1.54g) in ethanol. Anhydrous sodium acetate (0.78g) was added and the resulting mixture was stirred at reflux for 6 hours then allowed to stand at room temperature over the weekend. The solvent was evaporated. The residue was suspended in 2M hydrochloric acid and extracted with ethyl acetate. The organic extract was washed with aqueous sodium bicarbonate then water then dried and evaporated. The resulting red solid was triturated with ether/cyclohexane then further purified by column chromatography (dichloromethane eluent). Further trituration of the resulting product with cyclohexane yielded 3-chloro-8-(2,3-difluoromethylenedioxyphenyl)-6-(4-fluorophenyl)pyrido[2,3-d]pyridazin-5-one (compound 7, 1.29g) as a peach solid, m.p. 164-165^{o}C.

By proceeding in a similar manner the following compound was prepared:-
8-(2,3-difluoromethylenedioxyphenyl-3-fluoro-6-(4-fluorophenyl)pyrido[2,3-d]pyridazin-5-one (compound 13), m.p. 159-161^{o}C.

### Example 3

2-(2,3-Difluoromethylenedioxybenzoyl)nicotinic acid (6.14g) was added to a solution of 4-trifluoromethylphenylhydrazine (3.52g) in toluene and the mixture was stirred at reflux temperature for 4 hours. Water was removed azeotropically using a Dean-Stark apparatus. The reaction mixture was washed successively with 2N hydrochloric acid, saturated sodium hydrogen carbonate and brine. The organic phase was dried and the solvent evaporated. The crude product was triturated with ether/hexane to yield 8-(2,3-difluoromethylenedioxyphenyl)-6-(4-trifluoromethylphenyl)pyrido[2,3-d]pyridazin-5-one (compound 3) as an orange solid (6.18g), m.p. 181.6-182^{o}C.

By proceeding in a similar manner (but replacing toluene with ethanol as solvent), the following compounds of formula I were prepared:-
6-(3,4-difluoromethylenedioxyphenyl)-8-(3-trifluoromethylphenyl)pyrido[2,3-d]pyridazin-5-one (compound 6), m.p. 102.5-106^{o}C;
3-chloro-8-(2,3-difluoromethylenedioxyphenyl)-6-(4-trifluoromethylphenyl)pyrido[2,3-d]pyridazin-5-one (compound 8), m.p. 186-187^{o}C;
6-(3-cyano-4-fluorophenyl)-8-(2,3-difluoromethylenedioxyphenyl)pyrido[2,3-d]pyridazin-5-one (compound 9), m.p. 209 -210.2^{o}C;
8-(2,3-difluoromethylenedioxyphenyl)-6-(4-fluoro-3-trifluoromethylphenyl)pyrido[2,3-d]pyridazin-5-one (compound 10), m.p. 177.6-178.6^{o}C;
6-(4-cyanophenyl)-8-(2,3-difluoromethylenedioxyphenyl)pyrido[2,3-d]pyridazin-5-one (compound 11), m.p. 213.4-214^{o}C;
8-(2,3-difluoromethylenedioxyphenyl)-6-(3,4-difluorophenyl)pyrido[2,3-d]pyridazin-5-one (compound 12), m.p. 158.5-159^{o}C;
8-(2,3-difluoromethylenedioxyphenyl)-3-fluoro-6-(4-trifluoromethylphenyl)pyrido[2,3-d]pyridazin-5-one (compound 14), m.p. 188-189^{o}C.

### Reference Example 1

A solution of 2,3-difluoromethylenedioxybenzoyl cyanide (30.2g) in dry 1,4-dioxane was added to a stirred suspension of sodium hydride (60% as an oil suspension, 18.24g) in dry 1,4-dioxane and stirred for 30 minutes. TDA-1 (0.44ml) was added and the reactions stirred for a further 5 minutes. A solution of 2-chloronicotinic acid (23.9g) in 1,4-dioxane was added. The reaction mixture was stirred at reflux temperature for 3 hours. The reaction mixture was allowed to cool to 70^{o}C and air was then passed through the stirred mixture for 3.5 hours, then left overnight at room temperature. Water was cautiously added and the reaction mixture was poured into water, washed with cyclohexane and filtered through "HiFlo" silica. The resulting solution was acidified to pH 2 with concentrated hydrochloric acid and extracted with ethyl acetate, washed with brine and water, dried and evaporated to yield 2-(2,3-difluoromethylenedioxybenzoyl)nicotinic acid (39.1g), m.p. 120-140^{o}C.

By proceeding in a similar manner the following compounds were prepared:-
2-(3,4-difluoromethylenedioxybenzoyl)nicotinic acid, NMR(CDCl₃): 7.07(d,1H), 7.38-7.6(m,3H), 8.35(dd,1H), 8.73(dd,1H);
2-(2,3-methylenedioxybenzoyl)nicotinic acid, m.p. 160-165^{o}C;
5-chloro-2-(2,3-difluoromethylenedioxybenzoyl)nicotinic acid, m.p. 115-120^{o}C;
2-(2,3-difluoromethylenedioxybenzoyl)-5-fluoronicotinic acid.

### Reference Example 2

2,3-Difluoromethylenedioxybenzyl bromide (40.4g) was dissolved in ethanol and potassium cyanide (11.3g) was added. The mixture was stirred at 70^{o}C for 5 hours. Water and a further portion of potassium cyanide (2g) were added and the mixture was stirred at 70^{o}C for a further 3 hours. Most of the solvent was removed in vacuo and water was added to the residue, which was extracted with diethyl ether, washed with water, dried and concentrated to yield 2,3-difluoromethylenedioxybenzyl cyanide as a yellow oil, (30.2g), NMR (CDCl₃): 3.80(s,2H), 7.10(m,3H).

By proceeding in a similar manner the following compounds were prepared;
3,4-difluoromethylenedioxybenzyl cyanide, NMR (CDCl₃): 3.63(s,2H), 7.0(s,3H);
2,3-methylenedioxybenzyl cyanide, m.p. 65.8-68.2^{o}C.

### Reference Example 3

Phosphorus tribromide (47g) was added to a solution of 2,3-difluoromethylenedioxybenzyl alcohol (32g) in diethyl ether at 0^{o}C. After 0.5 hours the reaction mixture was allowed to warm to ambient temperature and was stirred at ambient temperature for 3 hours. The reaction mixture was then cooled to 0^{o}C, methanol was added followed by water, the mixture was extracted with diethyl ether, separated and the organic layer washed with saturated sodium bicarbonate (until neutral), dried and the solvent removed to yield 2,3-difluoromethylenedioxybenzyl bromide as a pale yellow oil, (40.4g), NMR (CDCl₃): 4.50(s,2H), 7.10(m,3H).

By proceeding in a similar manner, the following compounds were prepared:-
3,4-difluoromethylenedioxybenzyl bromide, NMR (CDCl₃); 4.48(s,2H), 6.98-7.18(d of d/s,3H);
2,3-methylenedioxybenzyl bromide.

### Reference Example 4

A solution of sodium borohydride (5g) in methanol was added to a solution of 2,3-difluoromethylenedioxybenzaldehyde (36g) in methanol with cooling so that the temperature did not exceed 10^{o}C. The reaction mixture was then allowed to warm to ambient temperature and stirred for 1 hour. Most of the methanol was evaporated and the residue was poured into cold 20% sodium hydroxide solution, extracted with ether, separated and washed with brine (until neutral), dried and the solvent removed to yield 2,3-difluoromethylenedioxybenzyl alcohol as a colourless oil (33.7g), NMR (CDCl₃): 2.10(br,1H), 4.80(d,2H), 7.00(m,1H), 7.10(m,2H).

By proceeding in a similar manner, the following compound was prepared:-
3,4-difluoromethylenedioxybenzyl alcohol, NMR (CDCl₃): 2.15(br.s,1H), 4.66(s,2H), 6.95-7.16(m,3H).

### Reference Example 5

A solution of ethyl 2,3-methylenedioxybenzoate (5g) in diethyl ether was added to a suspension of lithium aluminium hydride (0.97g) in diethyl ether under an inert atmosphere. The resulting mixture was refluxed for 2 hours. A further quantity of lithium aluminium hydride (0.25g) was added and the mixture was refluxed for a further 1 hour. The mixture was cooled and the excess lithium aluminium hydride was decomposed by addition of ethyl acetate then 1M hydrochloric acid. The mixture was then separated, the organic phase was washed with 1 M hydrochloric acid then water, dried and the solvent evaporated to yield 2,3-methylenedioxybenzyl alcohol as a pale yellow oil (3.41g). NMR (CDCl₃) 4,7(s,2H), 5.95(s,2H), 6.76-6.87(m,3H).

### Reference Example 6

A solution of sodium nitrite (3g) in concentrated sulphuric acid (25ml) was added dropwise to a cooled (0^{o}C) solution of 3,4-(difluoromethylenedioxy)aniline (6.92g) in propionic acid (50ml) and concentrated sulphuric acid (5ml) such that the reaction temperature did not exceed 5^{o}C. The resulting mixture was stirred at 0-5^{o}C for a further 1 hour and then allowed to reach room temperature over a period of 1 hour. The mixture was recooled to 0^{o}C. A solution of stannous chloride dihydrate (28.8g) in concentrated hydrochloric acid (21ml) was then added to the stirred mixture so that the reaction temperature did not exceed 5^{o}C. The resulting mixture was stirred at room temperature for one hour and then diluted with 50% sodium hydroxide to pH 14. The mixture was extracted with diethyl ether. The organic extracts were washed with water, dried and evaporated to yield 3,4-(difluoromethylenedioxy)phenyl hydrazine as a dark oil (6.15g), NMR (CDCl₃) 3.6 (br.s,2H), 5.2(br.s,1H), 6.47(dd,1H), 6.65(d,1H), 6.88(d,1H).

By proceeding in a similar manner the following compounds were prepared:-
3-cyano-4-fluorophenylhydrazine, NMR (DMSO-d₆) 4.1 (br.s,2H), 7.0-7.38(m,4H);
4-fluoro-3-trifluoromethylphenylhydrazine, NMR (CDCl₃) 3.65(br.s,2H), 5.27(br.s,1H), 6.9-7.17(m,3H);
4-cyanophenylhydrazine, m.p. 67-76^{o}C;
3,4-difluorophenylhydrazine, NMR (CDCl₃) 3.57(br.s,2H), 5.15(br.s,1H), 6.5(m,1H), 6.7(m,1H), 7.0(m,1H).

### Reference Example 7

Stannous chloride dihydrate (39.5g) was added to a cooled mixture of 3,4-(difluoromethylenedioxy)nitrobenzene (11g), concentrated hydrochloric acid (66ml), water and tetrahydrofuran. The reaction temperature was not allowed to exceed 40^{o}C. The mixture was stirred at room temperature, under an inert atmosphere for 3 days. The mixture was diluted with water, basified with 50% sodium hydroxide and extracted with diethyl ether. The ether extracts were washed with brine, dried and evaporated to yield 3,4-(difluoromethylenedioxy)aniline as a dark oil (2.2g). NMR (CDCl₃) 3.65 (brs,2H), 6.3(dd,1H), 6.4(d,1H), 6.82(d,1H).

By proceeding in a similar manner, the following compound was prepared;
3-cyano-4-fluoroaniline, m.p. 99-100^{o}C.

### Reference Example 8

2-Chloro-5-nitronicotinic acid (3.3g) was added portionwise to a solution of stannous chloride (12.6g) in concentrated hydrochloric acid so that the reaction temperature did not exceed 40^{o}C. The mixture was then heated to 90^{o}C and maintained at that temperature for 1 hour. The mixture was allowed to cool to room temperature and water was added. The mixture was placed in a fridge overnight. The crystalline product was collected by filtration, washed with water then dried to yield 5-amino-2-chloronicotinic acid hydrochloride as a colourless solid (1.1g), NMR(DMSO-d₆) 7.0(br.s,2H), 7.6(s,1H), 7.95(s,1H).

### Reference Example 9

A solution of 3-bromo-2,5-dichloropyridine (10g) in diisopropyl ether was added dropwise to a stirred solution of n-butyllithium (2.5 M in hexanes, 17.7ml) in diisopropyl ether at -70^{o}C under an inert atmosphere so that the reaction temperature did not exceed -68^{o}C. An excess of solid carbon dioxide was then added to the suspension and the mixture was stirred for 2 hours (excess carbon dioxide evaporates, temperature reaches 10^{o}C.).

Ice/water was added, the mixture was stirred. The layers were separated. The aqueous layer was acidified to pH 2 with concentrated hydrochloric acid. The resulting precipitate was extracted into diethyl ether. The ether extracts were washed with water, dried (magnesium sulphate) and evaporated to yield a solid which was triturated in light petroleum to yield 2,5-dichloronicotinic acid as a cream solid (7.04g), m.p. 160-162^{o}C.

### Reference Example 10

5-Amino-2-chloronicotinic acid hydrochloride (3.8g) was dissolved in 50% tetrafluoroboric acid. Tetrahydrofuran was added and the resulting slurry was cooled to 0^{o}C. An aqueous solution of sodium nitrite (1.6g) was added and the reaction mixture was then maintained at 0-10^{o}C for a further 2 hours. The precipitate was collected by filtration, washed with cold (0-5^{o}C) ethanol then dried to yield 2-chloro-5-diazonium tetrafluoroborate nicotinic acid as a white solid (4.3g), m.p. 143-4^{o}C.

1,2-Dichlorobenzene was heated to 155^{o}C. The diazonium tetrafluoroborate salt (5g) prepared as described above was added portionwise as a slurry in 1,2-dichlorobenzene so that the reaction temperature was maintained at 150-155^{o}C. On completion of addition, the mixture was stirred at 150^{o}C for 15 minutes then allowed to cool to room temperature. The mixture was extracted with saturated sodium bicarbonate solution then water. The extracts were washed with diethyl ether and then acidified to pH 1 with concentrated hydrochloric acid. The mixture was extracted with ethyl acetate. The organic extracts were dried (magnesium sulphate) and evaporated to yield an orange gum which was triturated with hexane/diethyl ether to yield 2-chloro-5-fluoronicotinic acid as a yellow solid (1.4g), m.p. 123-8^{o}C.

### Reference Example 11

Dimethylformamide (4 drops) was added to a solution of 2-hydroxy-5-nitronicotinic acid (5g) in phosphorus oxychloride (10ml). The mixture was stirred at the reflux temperature for 3 hours. Excess solvent was removed by evaporation and the residue was then poured carefully into water, keeping the temperature of the resulting mixture at below 40^{o}C. The mixture was stirred at room temperature for a further 30 minutes then extracted with ethyl acetate. The extracts were washed with water, dried (magnesium sulphate) and evaporated. The resulting residue was triturated with ether/hexane to yield 2-chloro-5-nitronicotinic acid as a pale yellow solid (3.6g), m.p. 123-7^{o}C.

### Reference Example 12

Fuming nitric acid (26ml) was added to a stirred solution of 2-hydroxynicotinic acid (34.8g) in concentrated sulphuric acid (100ml) at 35-40^{o}C. The resulting mixture was then stirred at 50^{o}C for 4 hours. The mixture was cooled to room temperature and then poured onto ice. The resulting precipitate was collected by filtration, air-dried on the filter then recrystallised from ethanol to yield 2-hydroxy-5-nitronicotinic acid as a pale yellow solid (39.6g), m.p. 210-22^{o}C.

According to a further feature of the present invention, there are provided compositions suitable for herbicidal use comprising one or more of the pyrido[2,3-d]pyridazin-5-one and -5-thione derivatives of formula I or an agriculturally acceptable salt thereof, in association with, and preferably homogeneously dispersed in, one or more compatible agriculturally- acceptable diluents or carriers and/or surface active agents [i.e. diluents or carriers and/or surface active agents of the type generally accepted in the art as being suitable for use in herbicidal compositions and which are compatible with compounds of formula I]. The term "homogeneously dispersed" is used to include compositions in which the compounds of formula I are dissolved in other components. The term "herbicidal compositions" is used in a broad sense to include not only compositions which are ready for use as herbicides but also concentrates which must be diluted before use. Preferably, the compositions contain from 0.05 to 90% by weight of one or more compounds of formula I.

The herbicidal compositions may contain both a diluent or carrier and surface-active (e.g. wetting, dispersing, or emulsifying) agent. Surface-active agents which may be present in herbicidal compositions of the present invention may be of the ionic or non-ionic types, e.g. sulphoricinoleates, quaternary ammonium derivatives, products based on condensates of ethylene oxide with alkyl and polyaryl phenols, e.g. nonyl- or octyl-phenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl- and dioctyl-sodium sulphonosuccinates and alkali and alkaline earth metal salts of high molecular weight sulphonic acid derivatives e.g. sodium and calcium lignosulphonates and sodium and calcium alkylbenzene sulphonates.

Suitably, the herbicidal compositions according to the present invention may comprise up to 10% by weight, e.g. from 0.05% to 10% by weight, of surface-active agent but, if desired, herbicidal compositions according to the present invention may comprise higher proportions of surface-active agent, for example up to 15% by weight in liquid emulsifiable suspension concentrates and up to 25% by weight in liquid water soluble concentrates.

Examples of suitable solid diluents or carriers are aluminium silicate, microfine silicon dioxide, talc, chalk, calcined magnesia, kieselguhr, tricalcium phosphate, powdered cork, adsorbent carbon black and clays such as kaolin and bentonite. The solid compositions (which may take the form of dusts, granules or wettable powders) are preferably prepared by grinding the compounds of formula I with solid diluents or by impregnating the solid diluents or carriers with solutions of the compounds of formula I in volatile solvents, evaporating the solvents and, if necessary, grinding the products so as to obtain powders. Granular formulations may be prepared by absorbing the compounds of formula I (dissolved in suitable solvents, which may, if desired, be volatile) onto the solid diluents or carriers in granular form and, if desired, evaporating the solvents, or by granulating compositions in powder form obtained as described above. Solid herbicidal compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, glycol ethers, tetrahydrofurfuryl alcohol, acetophenone, cyclohexanone, isophorone, N-alkyl pyrrolidones, toluene, xylene, mineral, animal and vegetable oils, esterified vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Powders, dispersible granules and liquid compositions in the form of concentrates may be diluted with water or other suitable diluents, for example mineral or vegetable oils, particularly in the case of liquid concentrates in which the diluent or carrier is an oil, to give compositions ready for use.

When desired, liquid compositions of the compound of formula I may be used in the form of self-emulsifying concentrates containing the active substances dissolved in the emulsifying agents or in solvents containing emulsifying agents compatible with the active substances, the simple addition of such concentrates to water producing compositions ready for use.

Liquid concentrates in which the diluent or carrier is an oil may be used without further dilution using the electrostatic spray technique.

Herbicidal compositions according to the present invention may also contain, if desired, conventional adjuvants such as adhesives, protective colloids, thickeners, penetrating agents, spreading agents, stabilisers, sequestering agents, anti-caking agents, colouring agents and corrosion inhibitors. These adjuvants may also serve as carriers or diluents.

Unless otherwise specified, the following percentages are by weight. Preferred herbicidal compositions according to the present invention are:
aqueous suspension concentrates which comprise from 10 to 70% of one or more compounds of formula I, from 2 to 10% of surface-active agent, from 0.1 to 5% of thickener and from 15 to 87.9% of water;
wettable powders which comprise from 10 to 90% of one or more compounds of formula I, from 2 to 10% of surface-active agent and from 8 to 88% of solid diluent or carrier;
water soluble or water dispersible powders which comprise from 10 to 90% of one or more compounds of formula I, from 2 to 40% of sodium carbonate and from 0 to 88% of solid diluent;
liquid water soluble concentrates which comprise from 5 to 50%, e.g. 10 to 30%, of one or more compounds of formula I, from 0 to 25% of surface-active agent and from 10 to 90%, e.g. 45 to 85%, of water miscible solvent, e.g. triethylene glycol, or a mixture of water-miscible solvent and water;
liquid emulsifiable suspension concentrates which comprise from 10 to 70% of one or more compounds of formula I, from 5 to 15% of surface-active agent, from 0.1 to 5% of thickener and from 10 to 84.9% of organic solvent, e.g. mineral oil;
water dispersible granules which comprise from 1 to 90%, e.g. 25 to 75% of one or more compounds of formula I, from 1 to 15%, e.g. 2 to 10%, of surface-active agent and from 5 to 95%, e.g. 20 to 60%, of solid diluent, e.g. clay, granulated with the addition of water to form a paste and then dried and
emulsifiable concentrates which comprise 0.05 to 90%, and preferably from 1 to 60% of one or more compounds of formula I, from 0.01 to 10%, and preferably from 1 to 10%, of surface-active agent and from 9.99 to 99.94%, and preferably from 39 to 98.99%, of organic solvent.

Herbicidal compositions according to the present invention may also comprise the compounds of formula I in association with, and preferably homogeneously dispersed in, one or more other pesticidally active compounds and, if desired, one or more compatible pesticidally acceptable diluents or carriers, surface-active agents and conventional adjuvants as hereinbefore described.

Examples of other pesticidally active compounds which may be included in, or used in conjunction with, the herbicidal compositions of the present invention include herbicides, for example to increase the range of weed species controlled for example alachlor [2-chloro-2,6'-diethyl-N-(methoxy-methyl)-acetanilide], atrazine [2-chloro-4-ethylamino-6-isopropylamino-1,3,5-triazine], bromoxynil [3,5-dibromo-4-hydroxybenzonitrile], chlortoluron [N'-(3-chloro-4-methylphenyl)-N,N-dimethylurea], cyanazine [2-chloro-4-(1-cyano-1- methylethylamino)-6-ethylamino-1,3,5-triazine], 2,4-D [2,4-dichlorophenoxy-acetic acid], dicamba [3,6-dichloro-2-methoxybenzoic acid], difenzoquat [1,2- dimethyl-3,5-diphenylpyrazolium salts], flampropmethyl [methyl N-2-(N- benzoyl-3-chloro-4-fluoroanilino)-propionate], fluometuron [N'-(3-trifluoromethylphenyl)-N,N-dimethylurea], isoproturon [N'-(4-isopropylphenyl)-N,N-dimethylurea], insecticides, e.g. synthetic pyrethroids, e.g. permethrin and cypermethrin, and fungicides, e.g. carbamates, e.g. methyl N-(1-butyl-carbamoyl- benzimidazol-2-yl)carbamate, and triazoles e.g. 1-(4-chloro-phenoxy)-3,3- dimethyl-1-(1,2,4-triazol-1-yl)-butan-2-one.

Pesticidally active compounds and other biologically active materials which may be included in, or used in conjunction with, the herbicidal compositions of the present invention, for example those hereinbefore mentioned, and which are acids, may, if desired, be utilized in the form of conventional derivatives, for example alkali metal and amine salts and esters.

According to a further feature of the present invention there is provided an article of manufacture comprising at least one of the pyrido[2,3-d]pyridazin-5-one and pyrido[2,3-d]pyridazin 5-thione derivatives of formula I or, as is preferred, a herbicidal composition as hereinbefore described, and preferably a herbicidal concentrate which must be diluted before use, comprising at least one of the pyrido[2,3-d]pyridazin-5-one and pyrido[2,3-d]pyridazin 5-thione derivativesof formula I within a container for the aforesaid derivative or derivatives of formula I, or a said herbicidal composition, and instructions physically associated with the aforesaid container setting out the manner in which the aforesaid derivative or derivatives of formula I or herbicidal composition contained therein is to be used to control the growth of weeds. The containers will normally be of the types conventionally used for the storage of chemical substances which are solid at normal ambient temperatures and herbicidal compositions particularly in the form of concentrates, for example cans and drums of metal, which may be internally lacquered, and plastics materials, bottles or glass and plastics materials and, when the contents of the container is a solid, for example granular, herbicidal compositions, boxes, for example of cardboard, plastics materials and metal, or sacks. The containers will normally be of sufficient capacity to contain amounts of the N-substituted pyrazole derivative or herbicidal compositions sufficient to treat at least one acre of ground to control the growth of weeds therein but will not exceed a size which is convenient for conventional methods of handling. The instructions will be physically associated with the container, for example by being printed directly thereon or on a label or tag affixed thereto. The directions will normally indicate that the contents of the container, after dilution if necessary, are to be applied to control the growth of weeds at rates of application between 0.01kg and 20kg of active material per hectare in the manner and for the purposes hereinbefore described.

The following Examples illustrate herbicidal compositions according to the present invention:

### EXAMPLE C1

A wettable powder is formed from :

| | |
|---|---|
| Active ingredient (compound 1) | 80% w/w |
| Sodium dodecylbenzene sulphonate | 3% w/w |
| Sodium N-methyl-N-oleyl taurate | 2% w/w |
| Sodium polycarboxylate | 1% w/w |
| Microfine silicon dioxide | 2% w/w |
| China clay | 12% w/w |

by blending the above ingredients and grinding the mixture in an air jet mill.

Similar wettable powders may be prepared as described above by replacing the pyrido[2,3-d]pyridazin-5-one derivative (compound 1) with other compounds of formula I.

### EXAMPLE C2

A suspension concentrate is formed from:

| | |
|---|---|
| Active ingredient (compound 1) | 60% w/v |
| Nonyl phenol 9 mole polyethoxylate | 0.5% w/v |
| Triethanolamine salt of phosphated tristyryl phenol 16 mole polyethoxylate | 1.5% w/v |
| Sodium polycarboxylate | 0.4% w/v |
| polysaccharide gum | 0.1% w/v |
| propylene glycol | 5% w/v |
| silicone antifoam emulsion | 0.01% w/v |
| 1,2-benzisothiazolin-3-one solution in diipropylene glycol | 0.01% w/v |
| water | to 100 volumes |

by mixing using a high shear mixer all ingredients into 90% volume of water, then making up to volume with water, then milling the mixture by passing through a horizontal bead mill.

Similar suspension concentrates may be prepared as described above by replacing the pyrido[2,3-d]pyridazin-5-one derivative (compound 1) with other compounds of formula I.

### EXAMPLE C3

A granule is formed from

| | |
|---|---|
| Active ingredient (compound 1) | 5% w/w |
| Sepiolite granules 30/60 mesh | 95% w/w |

by dissolving the active ingredient in n-butanol, then spraying this solution onto sepiolite granules whilst mixing the granules in a tumbler-mixer then evaporating off the n-butanol to leave a granule containing 5% w/w active ingredient.

Similar granules may be prepared as described above by replacing the pyrido[2,3-d]pyridazin-5-one derivative (compound 1) with other compounds of formula I.

### EXAMPLE C4

A water dispersible granule is formed from

| | |
|---|---|
| Active ingredient (compound 1) | 75% w/w |
| Sodium lignosulphonate | 10% w/w |
| Sodium dialkylnaphthalene sulphonate | 3% w/w |
| Clay | 12% w/w |

by blending the above ingredients, then grinding the mixture in an air-jet mill, then adding water to form a kneadable paste, then extruding this paste to form fine filaments approximately 1mm in diameter, chopping the extrudate into lengths of approximately 4mm then drying these in a fluid bed drier.

Similar water dispersible granules may be prepared as described above by replacing the pyrido[2,3-d]pyridazin-5-one derivative (compound 1) with other compounds of formula I.

According to a feature of the present invention, there is provided a method for controlling the growth of weeds (i.e. undesired vegetation) at a locus which comprises applying to the locus a herbicidally effective amount of at least one pyrido[2,3-d]pyridazin-5-one or pyridazin-5-thione derivative of formula (I) or an agriculturally acceptable salt thereof. For this purpose, the pyrido[2,3-d]pyridazin-5-one or pyridazin-5-thione derivatives are normally used in the form of herbicidal compositions (i.e. in association with compatible diluents or carriers and/or surface active agents suitable for use in herbicidal compositions), for example as hereinafter described.

The compounds of formula (I) show herbicidal activity against dicotyledonous (i.e. broad-leafed) and monocotyledonous (e.g. grass) weeds by pre- and/or post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of formula (I) may be used to control the growth of:
broad-leafed weeds, for example, Abutilon theophrasti, Amaranthus retroflexus, Bidens pilosa, Chenopodium album, Galium aparine, Ipomoea spp. e.g. Ipomoea purpurea, Sesbania exaltata, Sinapis arvensis, Solanum nigrum and Xanthium strumarium, and
grass weeds, for example Alopecurus myosuroides, Avena fatua, Digitaria sanguinalis, Echinochloa crus-galli, Eleusine indica and Setaria spp, e.g. Setaria faberii or Setaria viridis, and
sedges, for example, Cyperus esculentus.

The amounts of compounds of formula (I) applied vary with the nature of the weeds, the compositions used, the time of application, the climatic and edaphic conditions and (when used to control the growth of weeds in crop-growing areas) the nature of the crops. When applied to a crop-growing area, the rate of application should be sufficient to control the growth of weeds without causing substantial permanent damage to the crop. In general, taking these factors into account, application rates between 0.01kg and 5kg of active material per hectare give good results. However, it is to be understood that higher or lower application rates may be used, depending upon the particular problem of weed control encountered.

The compounds of formula (I) may be used to control selectively the growth of weeds, for example to control the growth of those species hereinbefore mentioned, by pre- or post-emergence application in a directional or non-directional fashion, e.g. by directional or non-directional spraying, to a locus of weed infestation which is an area used, or to be used, for growing crops, for example cereals, e.g. wheat, barley, oats, maize and rice, soya beans, field and dwarf beans, peas, lucerne, cotton, peanuts, flax, onions, carrots, cabbage, oilseed rape, sunflower, sugar beet, and permanent or sown grassland before or after sowing of the crop or before or after emergence of the crop. For the selective control of weeds at a locus of weed infestation which is an area used, or to be used, for growing of crops, e.g. the crops hereinbefore mentioned, application rates between 0.01kg and 4.0kg, and preferably between 0.01kg and 2.0kg, of active material per hectare are particularly suitable. The use of the compounds for controlling weeds in cereal crops (e.g. wheat and barley) is particularly preferred.

The compounds of formula (I) may also be used to control the growth of weeds, especially those indicated above, by pre- or post-emergence application in established orchards and other tree-growing areas, for example forests, woods and parks, and plantations, e.g. sugar cane, oil palm and rubber plantations. For this purpose they may be applied in a directional or non- directional fashion (e.g. by directional or non-directional spraying) to the weeds or to the soil in which they are expected to appear, before or after planting of the trees or plantations at application rates between 0.25kg and 5.0kg, and preferably between 0.5kg and 4.0kg of active material per hectare.

The compounds of formula (I) may also be used to control the growth of weeds, especially those indicated above, at loci which are not crop-growing areas but in which the control of weeds is nevertheless desirable.

Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The precise dosage will depend upon the nature of the vegetation treated and the effect sought.

Pre- or post-emergence application, and preferably pre-emergence application, in a directional or non-directional fashion (e.g. by directional or non-directional spraying) at application rates between 1.0kg and 20.0kg, and preferably between 5.0 and 10.0kg, of active material per hectare are particularly suitable for this purpose.

When used to control the growth of weeds by pre-emergence application, the compounds of formula (I) may be incorporated into the soil in which the weeds are expected to emerge. It will be appreciated that when the compounds of formula (I) are used to control the growth of weeds by post-emergence application, i.e. by application to the aerial or exposed portions of emerged weeds, the compounds of formula (I) will also normally come into contact with the soil and may also then exercise a pre-emergence control on later-germinating weeds in the soil.

Where especially prolonged weed control is required, the application of the compounds of formula (I) may be repeated if required.

Representative compounds of formula (I) have been used in herbicidal applications according to the following procedures.

### METHOD OF USE OF HERBICIDAL COMPOUNDS:

### a) General

Appropriate quantities of the compounds used to treat the plants were dissolved in acetone to give solutions equivalent to application rates of up to 4000g test compound per hectare (g/ha). These solutions were applied from a standard laboratory herbicide sprayer delivering the equivalent of 290 litres of spray fluid per hectare.

### b) Weed control : Pre-emergence

The seeds were sown in 70 mm square, 75 mm deep plastic pots in non-sterile soil. The quantities of seed per pot were as follows:-

| Weed species | Approx number of seeds/pot |
|---|---|
| 1) Broad-leafed weeds | |
| Abutilon theophrasti | 10 |
| Amaranthus retroflexus | 20 |
| Galium aparine | 10 |
| Ipomoea purpurea | 10 |
| Sinapis arvensis | 15 |
| Xanthium strumarium | 2. |

| 2) Grass weeds | |
|---|---|
| Alopecurus myosuroides | 15 |
| Avena fatua | 10 |
| Echinochloa crus-galli | 15 |
| Setaria viridis | 20. |

| 3) Sedges | |
|---|---|
| Cyperus esculentus | 3. |

| Crop | |
|---|---|
| 1) Broad-leafed | |
| Cotton | 3 |
| Soya | 3. |

| 2) Grass | |
|---|---|
| Maize | 2 |
| Rice | 6 |
| Wheat | 6. |

The compounds of the invention were applied to the soil surface, containing the seeds, as described in (a). A single pot of each crop and each weed was allocated to each treatment, with unsprayed controls and controls sprayed with acetone alone.

After treatment the pots were placed on capillary matting kept in a glass house, and watered overhead. Visual assessment of crop damage was made 20-24 days after spraying. The results were expressed as the percentage reduction in growth or damage to the crop or weeds, in comparison with the plants in the control pots.

### c) Weed control: Post-emergence

The weeds and crops were sown directly into John Innes potting compost in 75 mm deep, 70 mm square pots except for Amaranthus which was pricked out at the seedling stage and transferred to the pots one week before spraying. The plants were then grown in the greenhouse until ready for spraying with the compounds used to treat the plants. The number of plants per pot were as follows :-

| 1) Broad leafed weeds | | |
|---|---|---|
| Weed species | Number of plants per pot | Growth stage |
| Abutilon theophrasti | 3 | 1-2 leaves |
| Amaranthus retroflexus | 4 | 1-2 leaves |
| Galium aparine | 3 | 1^{st} whorl |
| Ipomoea purpurea | 3 | 1-2 leaves |
| Sinapis arvensis | 4 | 2 leaves |
| Xanthium strumarium | 1 | 2-3 leaves. |

| 2) Grass weeds | | |
|---|---|---|
| Weed species | Number of plants per pot | Growth stage |
| Alopecurus myosuroides | 8-12 | 1-2 leaves |
| Avena fatua | 12-18 | 1-2 leaves |
| Echinochloa crus-galli | 4 | 2-3 leaves |
| Setaria viridis | 15-25 | 1-2 leaves. |

| 3) Sedges | | |
|---|---|---|
| Weed species | Number of plants per pot | Growth stage |
| Cyperus esculentus | 3 | 3 leaves. |

| 1) Broad leafed | | |
|---|---|---|
| Crops | Number of plants per pot | Growth stage |
| Cotton | 2 | 1 leaf |
| Soya | 2 | 2 leaves. |

| 2) Grass | | |
|---|---|---|
| Crops | Number of plants per pot | Growth stage |
| Maize | 2 | 2-3 leaves |
| Rice | 4 | 2-3 leaves |
| Wheat | 5 | 2-3 leaves. |

The compounds used to treat the plants were applied to the plants as described in (a). A single pot of each crop and weed species was allocated to each treatment, with unsprayed controls and controls sprayed with acetone alone.

After treatment the pots were placed on capillary matting in a glass house, and watered overhead once after 24 hours and then by controlled sub-irrigation. Visual assessment of crop damage and weed control was made 20-24 days after spraying. The results were expressed as the percentage reduction in growth or damage to the crop or weeds, in comparison with the plants in the control pots.

When applied at 1000g/hectare pre- or post-emergence, compounds 1 to 14 gave at least 90% reduction in growth of one or more weed species.

## Claims

1. A method of controlling the growth of weeds at a locus which comprises applying to the locus a herbicidally effective amount of a pyrido[2,3-d]pyridazin-5-one or -5-thione derivative of formula (I): 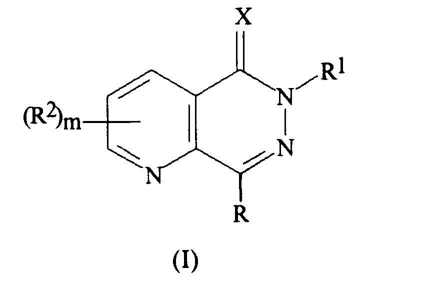 wherein:-
R and R¹, which may be the same or different, each represents:-
the hydrogen atom,
a straight- or branched chain alkyl, alkenyl or alkynyl group containing up to six carbon atoms optionally substituted by one or more halogen atoms;
a group -(-CR³R⁴)ₙ-(phenyl)-(R⁵)_{q};
a group -(-CR³R⁴)ₙHet;
a group -(-CR³R⁴)ₙ-Ar, wherein Ar represents phenyl or pyridyl optionally substituted by one or more groups R⁵ and wherein two substituents on adjacent positions of the ring, together with the two atoms to which they are attached, form a 5- to 7-membered alicyclic ring (which is optionally unsaturated) or an aromatic ring, optionally containing one or more heteroatoms, wherein the alicyclic or aromatic ring is optionally substituted by one or more groups R⁵¹ which may be the same or different;
provided that at least one of the groups R and R¹ represents -(-CR³R⁴)ₙ-Ar;
R² represents:-
a group R⁵;
or phenyl optionally substituted by from one to five groups R⁵ which may be the same or different;
X represents oxygen or sulphur;
m represents zero or an integer from one to three;
where m is greater than one the groups R² may be the same or different;
R³ and R⁴, which may be the same or different, each represents the hydrogen atom or a straight- or branched- chain alkyl group containing up to four carbon atoms optionally substituted by one or more halogen atoms;
R⁵ represents:-
a halogen atom;
a straight- or branched- chain alkyl, alkenyl or alkynyl group containing up to six carbon atoms optionally substituted by one or more halogen atoms; or
a group selected from cyano, nitro, -CO₂R⁶, -S(O)ₚR⁶, -NR³R⁴, -COR⁶, -S(O)ₚR⁷, -CO₂R⁷, -OR⁷, -CONR³R⁴, -OSO₂R⁷, -OSO₂R⁸, -OCH₂R⁷, -N(R³)COR⁸, -N(R³)SO₂R⁸, -N(R³)SO₂R⁷, -SO₂NR³R⁴, -Si(R⁸)₃ and -OR⁶;
n represents zero, one or two; where n is two the groups -(-CR³R⁴)- may be the same or different;
q represents zero or an integer from one to five; where q is greater than one the groups R⁵ may be the same or different;
R⁵¹ is as defined above for R⁵ or represents =O, =S or a group -O(-CR⁶¹R⁶²-)ᵣ-O-;
Het represents a 5- or 6- membered heterocycle containing from 3 to 5 carbon atoms in the ring and from 1 to 3 heteroatoms in the ring selected from nitrogen, sulphur and oxygen, optionally substituted by one or more groups R⁵ which may be the same or different;
R⁶ represents the hydrogen atom or a straight- or branched-chain alkyl group containing up to six carbon atoms optionally substituted by one or more halogen atoms;
p represents zero, one or two;
r represents one or two; where r is two the groups -(-CR⁶¹R⁶²)- may be the same or different;
R⁷ represents phenyl optionally substituted by from one to five groups which may be the same or different selected from halogen, nitro, cyano, R⁶ and -OR⁶;
R⁸ represents a straight- or branched- chain alkyl group containing up to six carbon atoms optionally substituted by one or more halogen atoms;
R⁶¹ and R⁶², which may be the same or different, each represents a hydrogen or halogen atom or a straight- or branched-chain alkyl group containing up to four carbon atoms optionally substituted by one or more halogen atoms;
or an agriculturally acceptable salt thereof.

2. A method according to claim 1 in which the locus is an area used, or to be used, for growing of crops and the compound is applied at an application rate from 0.01 kg to 4.0 kg per hectare.

3. A method according to claim 1 or 2 in which the locus is an area used for the growth of cereal crops.

4. A compound of formula (I) as defined in claim 1 or an agriculturally acceptable salt thereof, with the proviso that when X is oxygen, m is zero and R represents 3,4-methylenedioxyphenyl, R¹ is not selected from the group consisting of hydrogen, phenyl, benzyl, ethyl and 4-pyridylmethyl.

5. A compound according to claim 4 wherein:-
R represents a group -(-CR³R⁴)ₙ-Ar, wherein Ar represents phenyl optionally substituted by from one to three groups R⁵ and wherein two substituents in the 2,3- or 3,4- positions of the ring, together with the two atoms to which they are attached, form a 5- or 6- membered alicyclic ring (which is optionally unsaturated) or an aromatic ring, containing one or two heteroatoms selected from oxygen, sulphur and nitrogen, wherein the alicyclic or aromatic ring is optionally substituted by one or more groups R⁵¹ which may be the same or different;
R¹ represents phenyl optionally substituted by from one to five groups R⁵ which may be the same or different;
R² represents:-
a halogen atom;
a straight- or branched- chain alkyl, alkenyl or alkynyl group containing up to six carbon atoms optionally substituted by one or more halogen atoms; or
a group selected from cyano, nitro, -CO₂R⁶, -S(O)ₚR⁶, -NR³R⁴, -COR⁶ and -OR⁶;
n represents zero;
R⁵¹ represents:-
a halogen atom;
a straight- or branched- chain alkyl group containing up to six carbon atoms optionally substituted by one or more halogen atoms;
a group selected from cyano, nitro, -CO₂R⁶, S(O)ₚR⁶, -NR³R⁴, -COR⁶, -S(O)ₚR⁷, -CO₂R⁷, -OR⁷, -CONR³R⁴, -OSO₂R⁷ and -OR⁶; and
X represents oxygen.

6. A compound according to claim 4 or 5 wherein m is one and R² occupies the 3-position of the pyridyl ring.

7. A compound according to claim 4, 5 or 6 wherein
R represents 2,3-methylenedioxyphenyl, wherein the methylene group is optionally substituted by one or two groups R⁵¹ which may be the same or different;
R¹ represents phenyl optionally substituted in the 3- and/or 4-position by R⁵;
R² represents:-
a halogen atom;
a straight- or branched chain alkyl group containing up to four carbon atoms optionally substituted by one or more halogen atoms;
or a group selected from -S(O)ₚR⁶, -NR³R⁴ and -OR⁶;
R⁵¹ represents:-
a halogen atom; or
a straight- or branched chain alkyl group containing up to four carbon atoms optionally substituted by one or more halogen atoms;
R⁵ represents:-
a halogen atom; a cyano group; or
a straight- or branched chain alkyl group containing up to four carbon atoms optionally substituted by one or more halogen atoms;
m represents zero, one or two; and
X represents oxygen.

8. A compound according to claim 4 wherein:
R and R¹, which may be the same or different, each represents:-
phenyl substituted in the 3- and/or 4- position by R⁵;
2,3- or 3,4-methylenedioxyphenyl, wherein the methylene group is optionally substituted by two fluorine atoms;
provided that at least one of the groups R and R¹ is 2,3- or 3,4-methylenedioxyphenyl, wherein the methylene group is optionally substituted by two fluorine atoms;
R² represents a halogen atom;
R⁵ represents a halogen atom, trifluoromethyl or cyano;
m represents zero or one; and
X represents oxygen.

9. A compound according to claim 4 which is:
8-(2,3-difluoromethylenedioxyphenyl)-6-(4-fluorophenyl)pyrido[2,3-d]pyridazin-5-one;
6-(4-chlorophenyl)-8-(2,3-difluoromethylenedioxyphenyl)pyrido[2,3-d]pyridazin-5-one;
8-(2,3-difluoromethylenedioxyphenyl)-6-(4-trifluoromethylphenyl)pyrido[2,3-d]pyridazin-5-one;
8-(3,4-difluoromethylenedioxyphenyl)-6-(4-fluorophenyl)pyrido[2,3-d]pyridazin-5-one;
6-(4-fluorophenyl)-8-(2,3-methylenedioxyphenyl)pyrido[2,3-d]pyridazin-5-one;
6-(3,4-difluoromethylenedioxyphenyl)-8-(3-trifluoromethylphenyl)pyrido[2,3-d]pyridazin-5-one;
3-chloro-8-(2,3-difluoromethylenedioxyphenyl)-6-(4-fluorophenyl)pyrido[2,3-d]pyridazin-5-one;
3-chloro-8-(2,3-difluoromethylenedioxyphenyl)-6-(4-trifluoromethylphenyl)pyrido[2,3-d]pyridazin-5-one;
6-(3-cyano-4-fluorophenyl)-8-(2,3-difluoromethylenedioxyphenyl)pyrido[2,3-d]pyridazin-5-one;
8-(2,3-difluoromethylenedioxyphenyl)-6-(4-fluoro-3-trifluoromethylphenyl)pyrido[2,3-d]pyridazin-5-one;
6-(4-cyanophenyl)-8-(2,3-difluoromethylenedioxyphenyl)pyrido[2,3-d]pyridazin-5-one;
8-(2,3-difluoromethylenedioxyphenyl)-6-(3,4-difluorophenyl)pyrido[2,3-d]pyridazin-5-one;
8-(2,3-difluoromethylenedioxyphenyl)-3-fluoro-6-(4-fluorophenyl)pyrido[2,3-d]pyridazin-5-one; or
8-(2,3-difluoromethylenedioxyphenyl)-3-fluoro-6-(4-trifluoromethylphenyl)pyrido[2,3-d]pyridazin-5-one;
or an agriculturally acceptable salt thereof.

10. A composition suitable for herbicidal use comprising as active ingredient a herbicidally effective amount of a compound of formula (I) as defined in any one of claims 4 to 9 or an agriculturally acceptable salt thereof, in association with one or more compatible agriculturally- acceptable diluents or carriers.

11. A composition according to claim 10 comprising from about 0.05% to about 95% by weight of active ingredient and from about 0.05 to about 25% by weight of surface active agent.

12. A composition according to claim 10 or 11 which is in the form of an aqueous suspension concentrate, a wettable powder, a soluble powder, a liquid water soluble concentrate, a liquid emulsifiable suspension concentrate, a granule or an emulsifiable concentrate.

13. A process for the preparation of a compound of formula I as defined in claim 1, said process comprising:
(a) reacting a compound of formula II: 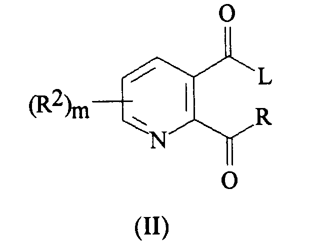 wherein R, R² and m are as defined in claim 1 and L is a leaving group, with a hydrazine of formula III or a salt thereof:
R¹-NHNH₂ (III)
wherein R¹ is as defined in claim 1;
(b) where X represents oxygen, cyclising a compound of formula (IIa): 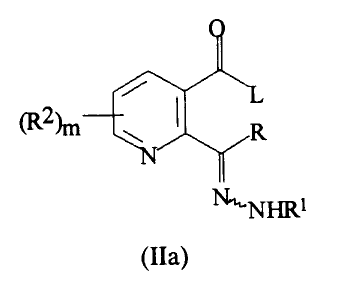 wherein R, R¹, R² and m are as defined in claim 1 and L is as defined above;
(c) where X is sulphur, treating the corresponding compound of formula I in which X represents oxygen with a thionation reagent to convert the carbonyl group to a thiocarbonyl group;
optionally followed by converting the compound of formula I thus obtained into an agriculturally acceptable salt thereof.

14. A compound useful as an intermediate in the preparation of a compound of fomula I as defined in claim 1, wherein said compound is of formula (II): 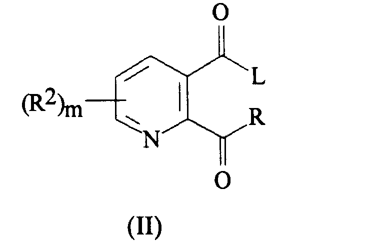 or of formula (IIa): 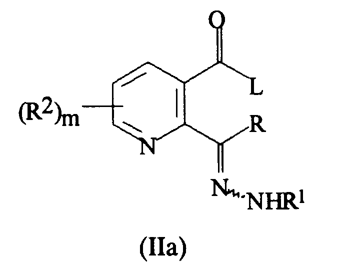 wherein R, R¹, R² and m are as defined in claim 1 and L represents -OH, straight- or branched- chain alkoxy containing up to 4 carbon atoms, or halogen.
